# EUROPÄISCHE PATENTSCHRIFT

(11) **EP 1 177 759 B1**
(45) Veröffentlichungstag und Bekanntmachung des Hinweises auf die Patenterteilung: **20.10.2004**
(21) Anmeldenummer: 01116277.3
(22) Anmeldetag: 05.07.2001
(51) Int. Cl.: A61B 5/00, G01N 33/487

(54) **Mikrodialyseanordnung**
Microdialysis device
Dispositif de microdialyse

(30) Priorität: 04.08.2000 DE 10038835
(43) Veröffentlichungstag der Anmeldung: 06.02.2002
(73) Patentinhaber: Roche Diagnostics GmbH, 68305 Mannheim (DE); F. Hoffmann-La Roche AG, 4070 Basel (CH)
(72) Erfinder: Roeper, Josef, 67141 Neuhofen (DE); Schoemaker, Michael, 68163 Mannheim (DE); Hoerauf, Christian, 68723 Oftersheim (DE)
(74) Vertreter: Pfiz, Thomas, Dr.

(56) Entgegenhaltungen:
- EP-A- 0 367 752
- EP-A- 0 940 151
- WO-A-97/42868
- DE-A- 4 426 694
- FR-A- 1 460 954

## Beschreibung

Die Erfindung betrifft eine Mikrodialyseanordnung mit einer in organisches Gewebe einsetzbaren, eine Dialysemembran zur Trennung eines mit Perfusionsflüssigkeit beaufschlagten Sondenkanals gegenüber dem Gewebe aufweisenden Mikrodialysesonde, einer Sensorzelle zur vorzugsweise elektrochemischen Erfassung von Inhaltsstoffen, insbesondere Glukose in der aus der Mikrodialysesonde abgeführten Perfusionsflüssigkeit und einer Fördereinrichtung zur Förderung der Perfusionsflüssigkeit durch den Sondenkanal der Mikrodialysesonde zu der Sensorzelle.

Bei einer aus der WO 97/42868 bekannten Meßanordnung dieser Art weist die Fördereinrichtung eine der im Körpergewebe eingebetteten Mikrodialysesonde nachgeordnete Dialysatpumpe auf, mittels welcher Perfusionsflüssigkeit aus einem Reservoir durch die Sonde hindurch angesaugt und unter Bildung von Dialysat an die extrakorporal angeordnete Sensorzelle weitergeleitet wird. Bei dieser Saugförderung ist der Betriebsdruck der Pumpe entsprechend des gewünschten Förderstroms zur Überwindung der Strömungshindemisse in dem Saugzweig eingestellt. Aufgrund der kleinlumigen Durchflußquerschnitte ergibt sich damit eine nennenswerte negative Druckdifferenz (Unterdruck) der Perfusionsflüssigkeit in dem Sondenkanal gegenüber der interstitiellen Flüssigkeit. Dies hat zur Folge, daß Gewebeflüssigkeit gleichsam durch Ultrafiltration über die Dialysemembran in die Sonde gesaugt wird. Bei *in vitro* Versuchen konnte sogar beobachtet werden, daß die gesamte Lösung am Auslaß der Sonde aus der Ultrafiltration stammt. Beim Saugbetrieb entspricht die tatsächliche Flußrate der die Mikrodialysesonde verlassenden Dialysatlösung zwar dem eingestellten Wert, aber die Herkunft der Flüssigkeit (Ultrafiltrat aus dem Körpergewebe oder Perfusat) ist ungewiß. Als weiterer Nachteil kommt hinzu, daß die Funktion der Dialysemembran durch ein negatives Druckgefälle beeinträchtigt werden kann. Insbesondere kann dies dazu führen, daß die aktive Austauschfläche durch gewebeseitige Anlagerung von Makromolekülen oder durch bauartbedingte Gegebenheiten reduziert und somit auch die Dialysatgewinnung verringert wird.

Umgekehrt stellt sich bei einer Druckförderung der Perfusionsflüssigkeit durch eine Perfusatpumpe im Zuleitungszweig der Mikrodialysesonde das Problem, daß aufgrund des erforderlichen Überdrucks Perfusionsflüssigkeit, also im wesentlichen Wasser über die Dialysemembran in das Gewebe austritt. Nachteilig wirkt sich dabei aus, daß die Gewebeflüssigkeit um die Sonde herum verdünnt wird, die Diffusion der Gewebeglucose in den Sondenkanal hinein im Gegenstrom gegen die Wassermoleküle erfolgen muß und die auslaßseitige Flußrate der Perfusionsflüssigkeit aufgrund des Flüssigkeitsverlusts unbestimmt ist. Ein weiterer Nachteil besteht darin, daß bei einem Ausfall der Pumpe die Gefahr besteht, daß im Auslaßzweig zudosierte Reagenzlösung über die Mikrodialysesonde eventuell in das Körpergewebe gelangt.

Ausgehend hiervon liegt der Erfindung die Aufgabe zugrunde, die genannten Nachteile zu beseitigen und eine Mikrodialyseanordnung der eingangs angegebenen Art dahingehend zu verbessern, daß eine zuverlässige und definierte Dialysefunktion gegeben ist.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Der Kern der Erfindung liegt darin, zur Optimierung der Diffusionsvorgänge über die Dialysemembran eine kombinierte Druck-Saug-Förderung der Perfusionsflüssigkeit vorzusehen. Dementsprechend wird erfindungsgemäß vorgeschlagen, daß die Fördereinrichtung eine druckseitig mit dem Einlaß des Sondenkanals verbundene Druckpumpeinheit sowie eine saugseitig mit dem Auslaß des Sondenkanals verbundene und simultan mit der Druckpumpeinheit betriebene Saugpumpeinheit aufweist. Damit läßt sich ein gewünschtes Druckniveau zwischen dem positiven Ausgangsdruck der Druckpumpeinheit und dem negativen Eingangsdruck der Saugpumpeinheit gezielt im Bereich der Dialysemembran einstellen. Vorteilhafterweise ist dabei die Druckpumpeinheit druckseitig ausschließlich mit dem Einlaß und die Saugpumpeinheit saugseitig ausschließlich mit dem Auslaß des Sondenkanals verbunden. Dies läßt sich dadurch realisieren, daß die Druckpumpeinheit und die Saugpumpeinheit über jeweils eine vorzugsweise durch einen Schlauch gebildete abzweigungsfreie Leitung mit dem Sondenkanal der Mikrodialysesonde verbunden sind. Auf diese Weise werden definierte Strömungsverhältnisse gewährleistet, und es wird sichergestellt, daß die Durchflußmenge durch jeden Durchflußquerschnitt in der jeweiligen Leitung in der gleichen Zeiteinheit gleich groß ist.

Eine bevorzugte Ausführung sieht vor, daß die Fördermengen der Druckund Saugpumpeinheit zur Reduzierung des über die Dialysemembran wirksamen Differenzdrucks zwischen Perfusions- und Gewebeflüssigkeit aufeinander abgestimmt und vorzugsweise im wesentlichen gleich sind. Damit wird ein Gleichgewicht auf niederem Druckniveau zwischen der Perfusionsflüssigkeit und der Gewebeflüssigkeit über die Dialysemembran erreicht, so daß keine Ultrafiltration auftritt und sowohl die Herkunft der Lösung, welche den Sondenkanal verläßt, als auch deren Fördermenge bzw. Flußrate bekannt und definiert ist. Dadurch wird auch sichergestellt, daß die Glucose allein durch Diffusion durch die Dialysemembran hindurchtritt. Zugleich wird eine Ansaugung der Membran an das Sondenlumen verhindert und somit die wirksame Membranfläche aufrecht erhalten.

Aufgrund des Druck-Saug-Betriebs ist es möglich, den Dialyseprozeß bei geringen Perfusionsgeschwindigkeiten ablaufen zu lassen. Vorteilhafterweise beträgt die Fördermenge der Druck- und Saugpumpeinheit weniger als 1 µl, vorzugsweise weniger als 0,1 µl in der Minute.

Im Langzeitbetrieb ist es günstig, wenn die Druckpumpeinheit saugseitig mit einem Reservoir für Perfusionsflüssigkeit verbunden ist, während die Saugpumpeinheit druckseitig mit einer vorzugsweise in einen Auffangbehälter mündenden Durchflußkammer der Sensorzelle verbunden ist. Die Sensor zelle bzw. Sensoreinheit weist dabei eine in die Durchflußkammer eingreifende elektrochemisch arbeitende Elektrodenanordnung auf, mit der in an sich bekannter Weise ein mit dem Glucosegehalt des Dialysats korreliertes Meßsignal erfaßbar ist. Grundsätzlich ist es auch möglich, daß die Saugpumpeinheit der Sensorzelle nachgeordnet ist, so daß die Durchflußkammer in die Saugstrecke einbezogen ist.

Zur chemischen Aufbereitung des Dialysats kann eine Reagenzpumpeinheit vorgesehen sein, mittels welcher sich eine Reagenzlösung, insbesondere Enzymlösung stromaufwärts der Sensorzelle in die Perfusionsflüssigkeit zudosieren läßt. Eine weitere Verbesserung auch im Hinblick auf die Verringerung des Kontaminationsrisikos wird dadurch erreicht, daß die Reagenzpumpeinheit und die Saugpumpeinheit druckseitig vorzugsweise über ein Y-Verbindungsstück an eine zu der Sensorzelle führende Verbindungsleitung angeschlossen sind.

Eine gerätetechnisch vorteilhafte Ausführung sieht vor, daß die Druckpumpeinheit, die Saugpumpeinheit und gegebenenfalls die Reagenzpumpeinheit durch jeweils einen über einen gemeinsamen Rollkolben betätigbaren Pumpschlauch einer mehrkanaligen Schlauchpumpe gebildet sind.

Vorteilhafterweise ist die Mikrodialysesonde durch einen doppellumigen Katheter gebildet, der im Bereich seines distalen Endes eine außenseitig in das Gewebe eingebettete und innenseitig mit Perfusionsflüssigkeit beaufschlagte, vorzugsweise als Hohlfaser ausgebildete mikroporige Dialysemembran aufweist.

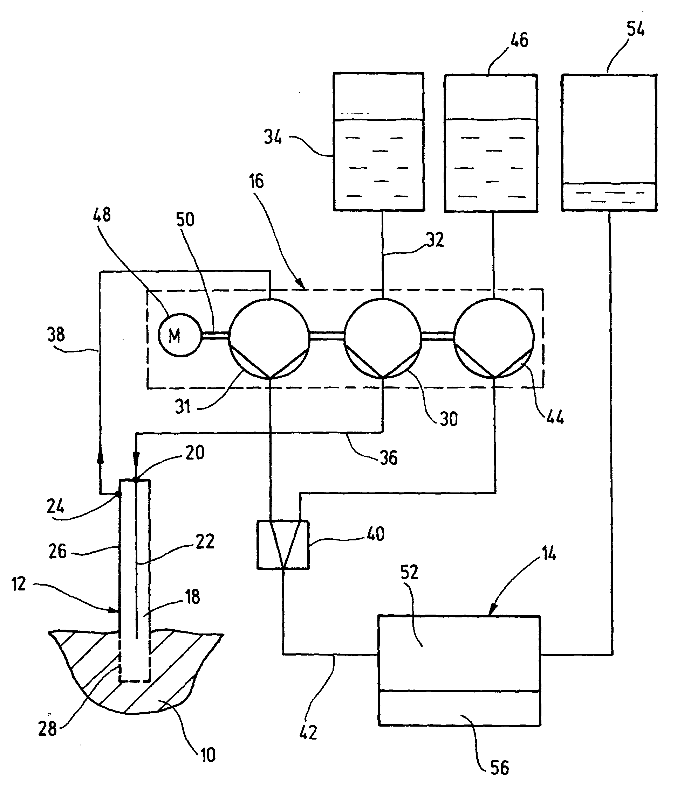

Im folgenden wird die Erfindung anhand eines in der Zeichnung in schematischer Weise dargestellten Ausführungsbeispiels näher erläutert. Die einzige Figur zeigt ein Blockschaltbild einer Mikrodialyseanordnung zur Konzentrationsbestimmung von Gewebeglucose.

Die in der Zeichnung dargestellte Mikrodialyseanordnung besteht als am Körper eines Patienten portables Meßsystem im wesentlichen aus einer in das Körpergewebe 10 einsetzbaren Mikrodialysesonde 12, einer der Mikrodialysesonde nachgeordneten Sensorzelle 14 und einer Fördereinrichtung 16 zum Transport von Perfusionsflüssigkeit durch die Mikrodialysesonde 12 hindurch zu der Sensorzelle 14.

Die Mikrodialysesonde 12 bildet als doppellumiger Katheter einen Sondenkanal 18, der über einen Einlaß 20 der Innenkanüle 22 und einen Auslaß 24 der konzentrischen Außenkanüle 26 durchströmbar ist, wobei die Innenkanüle 22 an ihrem distalen Ende mit der Außenkanüle 26 kommuniziert. In diesem Bereich ist eine als Hohlfaser ausgebildete Dialysemembran 28 angeordnet, welche den Sondenkanal 18 von dem umgebenden Gewebe 10 trennt und aufgrund ihrer mikroskopischen Porosität einen Diffusionsaustausch von Glucose zwischen der Gewebeflüssigkeit und der durch den Sondenkanal 18 hindurchgeleiteten Perfusionsflüssigkeit unter Gewinnung von Dialysat ermöglicht. Geeignete Mikrodialysesonden dieser Art sind insbesondere aus der DE-A 33 42 170 bzw. US-PS 4,694,832 bekannt und können von der in Solna, Schweden ansässigen Firma CMA/Microdialysis AB unter der Bezeichnung "CMA 60 Microdialysis Catheter" bzw. "CMA 70 Brain Microdialysis Catheter" erworben werden.

Zur kombinierten Druck- und Saugförderung der Perfusionsflüssigkeit weist die Fördereinrichtung 16 eine Druckpumpeinheit 30 und eine Saugpumpeinheit 31 auf. Die Druckpumpeinheit 30 ist saugseitig über eine Zuleitung 32 mit einem Perfusionsflüssigkeit, beispielsweise Ringer-Lösung enthaltenden Reservoir 34 und druckseitig über eine Druckleitung 36 mit dem Einlaß 20 des Sondenkanals 18 verbunden. Die Saugpumpeinheit 31 ist saugseitig über eine Saugleitung 38 mit dem Auslaß 24 des Sondenkanals 18 verbunden und speist die abgesaugte Perfusionsflüssigkeit druckseitig über ein Y-Verbindungsstück 40 in eine zu der Sensorzelle 14 führende Verbindungsleitung 42. Das Verbindungsstück 40 ermöglicht die Zumischung einer Reagenzlösung, insbesondere Enzymlösung zur enzymatisch katalysierten Oxidation der im Dialysat enthaltenen Gewebeglucose. Zu diesem Zweck weist die Fördereinrichtung 16 eine Reagenzpumpeinheit 44 auf, die saugseitig mit einem Reagenzlösungsreservoir 46 und druckseitig mit dem zweiten Anschluß des Verbindungsstücks 40 verbunden ist. Zweckmäßig sind die Druckpumpeinheit 30, die Saugpumpeinheit 31 und die Reagenzpumpeinheit 44 durch jeweils einen Pumpschlauch einer einzelnen Schlauchpumpe gebildet, so daß die Pumpschläuche über einen mittels Motor 48 angetriebenen Rollkolben 50 gemeinsam betätigbar sind. Grundsätzlich ist es auch möglich, mit immobilisierten Enzymen beschichtete Sensoren zu verwenden (vgl. DE-A-41 30 742), womit zwar auf die Zumischung einer Reagenzlösung verzichtet werden kann, jedoch Driftprobleme auftreten können.

Die Verbindungsleitung 42 mündet in eine Durchflußkammer 52 der Sensorzelle 14, welche ausgangsseitig mit einem Auffangbehälter 54 für die hindurchgeleitete Perfusionsflüssigkeit verbunden ist. Die elektrochemisch arbeitende Sensorzelle 14 weist eine in die Durchflußkammer 52 eingreifende Elektrodenanordnung 56 auf, deren Ausgangssignale als Maß für den Glukosegehalt des Dialysats an eine nicht gezeigte Auswerteeinrichtung übermittelbar sind. Einzelheiten der Meß- und Auswertetechnik sind beispielsweise aus der DE 44 01 400 A1 an sich bekannt. Anstelle einer elektrochemischen Meßzelle sind auch Detektoreinheiten oder ggf. Sammeleinheiten mit dezentraler Sensorik denkbar, die auf einer anderen Meßtechnik, beispielsweise optischer Nachweistechnik beruhen.

Die Pumpeinheiten 30, 31 sind über die Schlauchleitungen 36, 38 direkt, d.h. abzweigungsfrei mit der Mikrodialysesonde 12 verbunden. Dadurch ist ein simultaner Druck-Saug-Betrieb möglich, bei welchem die Perfusionsflüssigkeit unter getrennter Überwindung bzw. spezifischem Ausgleich der Strömungswiderstände der Druckleitung 36 und der Saugleitung 38 gefördert wird, wobei typischerweise die Leitungslänge im Bereich von einigen 10 cm und der Innendurchmesser bei 0,1 mm liegt. Die Perfusionsflüssigkeit läßt sich damit ohne Druckgefälle gegenüber der Gewebeflüssigkeit gleichsam drucklos an der Dialysemembran 28 vorbeiführen. Hierzu werden die Fördermengen der Pumpeinheiten 30, 31 und damit die ein- und auslaßseitigen Flußraten der Perfusionsflüssigkeit auf denselben Wert, beispielsweise 0,3 µl/min eingestellt. Auf diese Weise wird sichergestellt, daß kein ungewollter Flüssigkeitsdurchtritt durch die Dialysemembran 28 stattfindet, und daß eine definiert einstellbare Flüssigkeitsmenge in der Zeiteinheit die Mikrodialysesonde 12 durchströmt. Ein Stoffaustausch zwischen der Perfusionsflüssigkeit und dem die Mikrodialysesonde 12 umgebenden Gewebe 10 wird auf diese Weise im wesentlichen auf Diffusionsprozesse beschränkt und Flüssigkeitsströme zwischen Innen- und Außenraum werden weitestgehend vermieden. Soweit sich in einem einfachen Aufbau Förderdifferenzen zwischen Druckund Saugpumpe nicht vollständig vermeiden lassen, kann es vorteilhaft sein, wenn die Förderrate der Druckpumpe geringfügig über der Förderrate der Saugpumpe liegt, so daß eine ungewollte Ultrafiltration vermieden wird.

## Patentansprüche

1. Mikrodialyseanordnung mit einer in organisches Gewebe (10) einsetzbaren Mikrodialysesonde (12), welche eine Dialysemembran (28) zur Trennung eines mit Perfusionsflüssigkeit beaufschlagten Sondenkanals (18) gegenüber dem Gewebe (10) aufweist, einer Sensorzelle (14) zur vorzugsweise elektrochemischen Erfassung von Inhaltsstoffen, insbesondere Glukose in der aus der Mikrodialysesonde (12) abgeführten Perfusionsflüssigkeit und einer eine saugseitig mit dem Auslaß (24) des Sondenkanals (18) verbundene Saugpumpeinheit (31) aufweisenden Fördereinrichtung (16) zur Förderung der Perfusionsflüssigkeit durch den Sondenkanal (18) der Mikrodialysesonde (12) zu der Sensorzelle (14), **dadurch gekennzeichnet, daß** die Fördereinrichtung (16) weiterhin eine druckseitig mit dem Einlaß (20) des Sondenkanals (18) verbundene und simultan mit der Saugpumpeinheit (31) betriebene Druckpumpeinheit (30) aufweist.

2. Mikrodialyseanordnung nach Anspruch 1, **dadurch gekennzeichnet, daß** die Druckpumpeinheit (30) druckseitig ausschließlich mit dem Einlaß (20) und die Saugpumpeinheit (31) saugseitig ausschließlich mit dem Auslaß (24) des Sondenkanals (18) verbunden ist.

3. Mikrodialyseanordnung nach Anspruch 1 oder 2, **dadurch gekennzeichnet, daß** die Druckpumpeinheit (30) und die Saugpumpeinheit (31) über jeweils eine vorzugsweise durch einen Schlauch gebildete abzweigungsfreie Leitung (36,38) mit dem Sondenkanal (18) der Mikrodialysesonde verbunden sind.

4. Mikrodialyseanordnung nach einem der Ansprüche 1 bis 3, **dadurch gekennzeichnet, daß** die Fördermengen der Druck und Saugpumpeinheit (30,31) zur Reduzierung des über die Dialysemembran (28) wirksamen Differenzdrucks zwischen Perfusions- und Gewebeflüssigkeit aufeinander abgestimmt sind.

5. Mikrodialyseanordnung nach einem der Ansprüche 1 bis 4, **dadurch gekennzeichnet, daß** die Fördermengen der Druck- und Saugpumpeinheit (30,31) im wesentlichen gleich sind.

6. Mikrodialyseanordnung nach einem der Ansprüche 1 bis 4, **dadurch gekennzeichnet, daß** die Fördermenge der Druckpumpeinheit (30) um einen vorgegebenen Betrag größer ist als die Fördermenge der Saugpumpeinheit (31).

7. Mikrodialyseanordnung nach einem der Ansprüche 1 bis 6, **dadurch gekennzeichnet, daß** die Fördermenge der Druck- und Saugpumpeinheit (30,31) weniger als 1µl, vorzugsweise weniger als 0,1 µl in der Minute beträgt.

8. Mikrodialyseanordnung nach einem der Ansprüche 1 bis 7, **dadurch gekennzeichnet, daß** die Druckpumpeinheit (30) saugseitig mit einem Reservoir (34) für Perfusionsflüssigkeit verbunden ist.

9. Mikrodialyseanordnung nach einem der Ansprüche 1 bis 8, **dadurch gekennzeichnet, daß** die Saugpumpeinheit (31) druckseitig mit einer vorzugsweise in einen Auffangbehälter (54) mündenden Durchflußkammer (52) der Sensorzelle (14) verbunden ist.

10. Mikrodialyseanordnung nach einem der Ansprüche 1 bis 9, **gekennzeichnet durch** eine Reagenzpumpeinheit (44) zur Zudosierung einer Reagenzlösung, insbesondere Enzymlösung in die Perfusionsflüssigkeit stromaufwärts der Sensorzelle (14).

11. Mikrodialyseanordnung nach Anspruch 10, **dadurch gekennzeichnet, daß** die Reagenzpumpeinheit (44) und die Saugpumpeinheit (31) druckseitig vorzugsweise über ein Y-Verbindungsstück (40) an eine zu der Sensorzelle (14) führende Verbindungsleitung (42) angeschlossen sind.

12. Mikrodialyseanordnung nach einem der Ansprüche 1 bis 11, **dadurch gekennzeichnet, daß** die Druckpumpeinheit (30), die Saugpumpeinheit (31) und gegebenenfalls die Reagenzpumpeinheit (44) durch jeweils einen über einen gemeinsamen Rollkolben (50) betätigbaren Pumpschlauch einer mehrkanaligen Schlauchpumpe gebildet sind.

13. Mikrodialyseanordnung nach einem der Ansprüche 1 bis 12, **dadurch gekennzeichnet, daß** die Mikrodialysesonde (12) durch einen doppellumigen Katheter (22,26) gebildet ist, der im Bereich seines distalen Endes eine außenseitig in das Gewebe (10) eingebettete und innenseitig mit Perfusionsflüssigkeit beaufschlagte, vorzugsweise als Hohlfaser ausgebildete mikroporige Dialysemembran (28) aufweist.

## Claims

1. Microdialysis system comprising a microdialysis probe (12) that can be inserted in organic tissue (10) and has a dialysis membrane (28) to separate a probe channel (18) filled with perfusion fluid from the tissue (10), a sensor cell (14) for the preferably electrochemical detection of components and especially glucose in the perfusion fluid that is conveyed from the microdialysis probe (12) and a transport device (16) to convey the perfusion fluid through the probe channel (18) of the microdialysis probe (12) to the sensor cell (14), the transport device (16) having a suction pump unit (31) connected on the suction side to the outlet (24) of the probe channel (18), **characterized in that** the transport device (16) further has a pressure pump unit (30) connected on the pressure side to the inlet (20) of the probe channel (18) and being operated simultaneously with the suction pump unit (31).

2. Microdialysis system as claimed in claim 1, **characterized in that** the pressure side of the pressure pump unit (30) is exclusively connected to the inlet (20) and the suction side of the suction pump unit (31) is exclusively connected to the outlet (24) of the probe channel (18).

3. Microdialysis system as claimed in claim 1 or 2, **characterized in that** the pressure pump unit (30) and the suction pump unit (31) are each connected via a non-branching duct (36, 38), preferably comprising a flexible tube, to the probe channel (18) of the microdialysis probe.

4. Microdialysis system as claimed in one of the claims 1 to 3, **characterized in that** the delivery rates of the pressure and suction pump unit (30, 31) are matched in order to reduce the effective pressure difference across the dialysis membrane (28) between the perfusion and tissue fluid.

5. Microdialysis system as claimed in one of the claims 1 to 4, **characterized in that** the delivery rates of the pressure and suction pump unit (30, 31) are essentially equal.

6. Microdialysis system as claimed in one of the claims 1 to 4, **characterized in that** the delivery rate of the pressure pump unit (30) is larger by a predetermined amount than the delivery rate of the suction pump unit (31).

7. Microdialysis system as claimed in one of the claims 1 to 6, **characterized in that** the delivery rate of the pressure and suction pump unit (30, 31) is less than 1 µl, preferably less than 0.1 µl per minute.

8. Microdialysis system as claimed in one of the claims 1 to 7, **characterized in that** the suction side of the pressure pump unit (30) is connected to a reservoir (34) for perfusion fluid.

9. Microdialysis system as claimed in one of the claims 1 to 8, **characterized in that** the pressure side the suction pump unit (31) is connected to a flow chamber (52) of the sensor cell (14) which preferably terminates in a collecting vessel (54).

10. Microdialysis system as claimed in one of the claims 1 to 9, **characterized by** a reagent pump unit (44) for metering a reagent solution and in particular an enzyme solution into the perfusion fluid upstream of the sensor cell (14).

11. Microdialysis system as claimed in claim 10, **characterized in that** the pressure side of the reagent pump unit (44) and of the suction pump unit (31 ) are connected to a connecting duct (42) leading to the sensor cell (14) preferably via a Y connecting piece (40).

12. Microdialysis system as claimed in one of the claims 1 to 11, **characterized in that** the pressure pump unit (30), the suction pump unit (31) and optionally the reagent pump unit (44) each comprise a flexible pump tube of a multichannel peristaltic pump actuated by a common rotary piston (50).

13. Microdialysis system as claimed in one of the claims 1 to 12, **characterized in that** the microdialysis probe (12) comprises a double-lumen catheter (22, 26) which has in the area of its distal end a micropore dialysis membrane (28) formed from hollow fibres the outside of which is embedded in the tissue (10) and the inside of which is supplied with perfusion fluid.

## Revendications

1. Dispositif de microdialyse comportant une sonde de microdialyse (12) pouvant être insérée dans un tissu organique (10), laquelle sonde comprend une membrane de dialyse (28) servant à la séparation d'un canal de sonde (18), alimenté en liquide de perfusion, par rapport au tissu (10), une cellule sensorielle (14) servant à la détection de préférence électrochimique de composants, en particulier de glucose, dans le liquide de perfusion évacué de la sonde de microdialyse (12), et un dispositif de refoulement (16), servant au refoulement du liquide de perfusion à travers le canal (18) de la sonde de microdialyse (12) en direction de la cellule sensorielle (14), comportant une unité de pompe aspirante (31) raccordée côté aspiration à la sortie (24) du canal de sonde (18), **caractérisé en ce que** le dispositif de refoulement (16) comprend en outre une unité de pompe refoulante (30) raccordée côté pression à l'entrée (20) du canal de sonde (18) et actionnée en même temps que l'unité de pompe aspirante (31).

2. Dispositif de microdialyse selon la revendication 1, **caractérisé en ce que** l'unité de pompe refoulante (30) est raccordée côté pression exclusivement à l'entrée (20) et l'unité de pompe aspirante (31) est raccordée côté aspiration exclusivement à la sortie (24) du canal de sonde (18).

3. Dispositif de microdialyse selon la revendication 1 ou 2, **caractérisé en ce que** l'unité de pompe refoulante (30) et l'unité de pompe aspirante (31) sont raccordées au canal (18) de la sonde de microdialyse par le biais de, respectivement, une conduite (36, 38) sans embranchement formée de préférence par un tuyau souple.

4. Dispositif de microdialyse selon l'une quelconque des revendications 1 à 3, **caractérisé en ce que** les débits de l'unité de pompe refoulante et de l'unité de pompe aspirante (30, 31) sont adaptées l'une à l'autre pour la réduction de la pression différentielle, active par le biais de la membrane de dialyse (28), entre le liquide de perfusion et le liquide tissulaire.

5. Dispositif de microdialyse selon l'une quelconque des revendications 1 à 4, **caractérisé en ce que** les débits de l'unité de pompe refoulante et de l'unité de pompe aspirante (30, 31) sont identiques pour l'essentiel.

6. Dispositif de microdialyse selon l'une quelconque des revendications 1 à 4, **caractérisé en ce que** le débit de l'unité de pompe refoulante (30) dépasse, d'une valeur prédéfinie, le débit de l'unité de pompe aspirante (31).

7. Dispositif de microdialyse selon l'une quelconque des revendications 1 à 6, **caractérisé en ce que** le débit de l'unité de pompe refoulante et de l'unité de pompe aspirante (30, 31) est inférieur à 1 µl, de préférence inférieur à 0,1 µl à la minute.

8. Dispositif de microdialyse selon l'une quelconque des revendications 1 à 7, **caractérisé en ce que** l'unité de pompe refoulante (30) est raccordée côté aspiration à un réservoir (34) pour liquide de perfusion.

9. Dispositif de microdialyse selon l'une quelconque des revendications 1 à 8, **caractérisé en ce que** l'unité de pompe aspirante (31) est raccordée côté pression à une chambre de débit (52) de la cellule sensorielle (14), chambre débouchant de préférence dans un collecteur (54).

10. Dispositif de microdialyse selon l'une quelconque des revendications 1 à 9, **caractérisé par** une unité de pompe à réactif (44) pour l'addition dosée d'une solution réactive, en particulier d'une solution d'enzymes, dans le liquide de perfusion, en amont de la cellule sensorielle (14).

11. Dispositif de microdialyse selon la revendication 10, **caractérisé en ce que** l'unité de pompe à réactif (44) et l'unité de pompe aspirante (31) sont raccordées côté pression, de préférence par le biais d'une pièce de raccordement en Y (40), à une conduite de raccordement (42) conduisant à la cellule sensorielle (14).

12. Dispositif de microdialyse selon l'une quelconque des revendications 1 à 11, **caractérisé en ce que** l'unité de pompe refoulante (30), l'unité de pompe aspirante (31) et, le cas échéant, l'unité de pompe à réactif (44) sont formées par respectivement un tuyau de pompe, actionnable par le biais d'un piston rotatif (50) commun, d'une pompe à tuyau souple avec plusieurs canaux.

13. Dispositif de microdialyse selon l'une quelconque des revendications 1 à 12, **caractérisé en ce que** la sonde de microdialyse (12) est formée par un cathéter cloisonné (22, 26) qui comprend, au niveau de son extrémité distale, une membrane de dialyse (28) microporeuse, encastrée du côté externe dans le tissu (10) et alimentée du côté interne en liquide de perfusion, membrane réalisée de préférence sous la forme de fibres creuses.
